(19) Europäisches Patentamt
European Patent Office
Office européen des brevets

(11) EP 3 950 066 A1

(12) **EUROPEAN PATENT APPLICATION**
published in accordance with Art. 153(4) EPC

(43) Date of publication:
**09.02.2022 Bulletin 2022/06**

(21) Application number: **20784996.9**

(22) Date of filing: **26.03.2020**

(51) International Patent Classification (IPC):
***A61P 37/04** (2006.01)* ***A23L 33/10** (2016.01)*
***A61K 31/357** (2006.01)*

(52) Cooperative Patent Classification (CPC):
**A23L 33/10; A61K 31/357; A61P 37/04**

(86) International application number:
**PCT/JP2020/013504**

(87) International publication number:
**WO 2020/203593 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 JP 2019067663**

(71) Applicant: **Amino Up Co., Ltd.**
**Sapporo-shi**
**Hokkaido 004-0839 (JP)**

(72) Inventors:
- **ABE Keima**
  **Sapporo-shi, Hokkaido 004-0839 (JP)**
- **NISHIOKA Hiroshi**
  **Sapporo-shi, Hokkaido 004-0839 (JP)**
- **TAKANO Sho**
  **Sapporo-shi, Hokkaido 004-0839 (JP)**
- **TAKAHASHI Makiko**
  **Sapporo-shi, Hokkaido 004-0839 (JP)**
- **MATSUURA Hideyuki**
  **Sapporo-shi, Hokkaido 060-0808 (JP)**

(74) Representative: **Wasner, Marita**
**Niizuma Wasner GmbH**
**Patentanwaltskanzlei**
**Postfach 278**
**4125 Riehen (CH)**

(54) **IMMUNOSTIMULATOR AND FOOD OR BEVERAGE FOR IMMUNOSTIMULATION**

(57) An immunostimulator that comprises a compound represented by general formula I (wherein n stands for an integer of 4-12) or a pharmacologically acceptable salt thereof as an active ingredient.



Printed by Jouve, 75001 PARIS (FR)

(Cont. next page)

Fig. 3

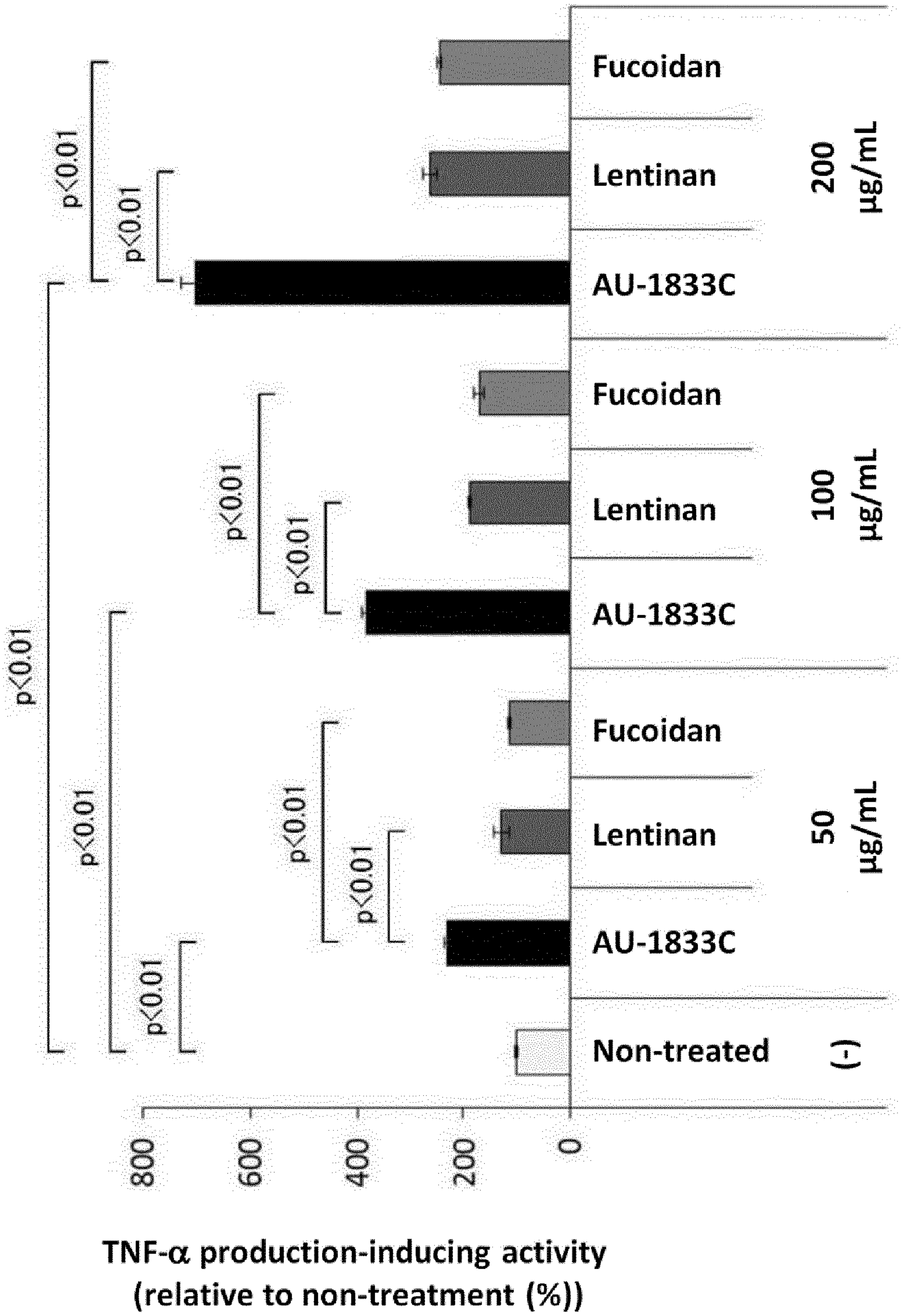

Fucoidan
Lentinan
AU-1833C
Fucoidan
Lentinan
AU-1833C
Fucoidan
Lentinan
AU-1833C
Non-treated
200 μg/mL
100 μg/mL
50 μg/mL
(-)
p<0.01
800
600
400
200
0
TNF-α production-inducing activity
(relative to non-treatment (%))

## Description

Technical Field

The present invention relates to an immunostimulator and a food or beverage for immunostimulation.

Background Art

Up to the present, various substances have been used as immunostimulators for enhancing the immune system. For example, Non Patent Literature 1 states that fucoidan has an immunostimulatory action.

Meanwhile, various physiological activities of carboxylate esters have been reported. For example, Patent Literature 1 discloses an antileishmania activity, and Patent Literature 2 discloses a pathogen controlling activity by plant pathogenic fungi.

Citation List

Patent Literature

Patent Literature 1: Japanese Patent Laid-Open No. 2016-160237
Patent Literature 2: Japanese Patent Laid-Open No. 2013-180995

Non Patent Literature

Non Patent Literature 1: Immune Efficacy and Safety of Fucoidan Extracted from Gagome Kombu° *(Kjellmaniella crassifolia)*°in Healthy Japanese Subjects, Hiromu Onogi et al., Japanese Journal of Complementary and Alternative Medicine, Vol. 12, Issue 2, 2015, p.87-93

Summary of Invention

Technical Problem

Development of a new immunostimulator has been long-awaited.

An object of the present invention is to provide a novel immunostimulator and a food or beverage for immunostimulation having an excellent immunostimulatory action.

Solution to Problem

The present inventors have found that a certain compound has an excellent immunostimulatory action, whereby the present invention is accomplished.

For achieving the above object, the immunostimulator according to the first aspect of the present invention comprises, as an active ingredient, a compound represented by general formula I:

[Formula 1]

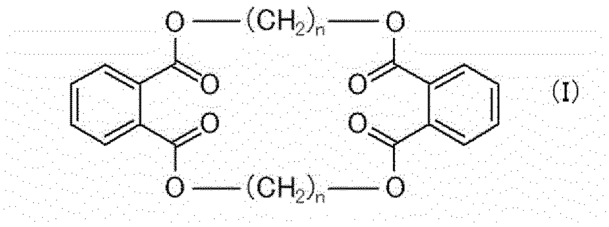

(I)

wherein n represents an integer of 4 to 12,

or a pharmacologically acceptable salt thereof.

For example, the immunostimulator comprises, as an active ingredient, a compound represented by formula II:

[Formula 2]

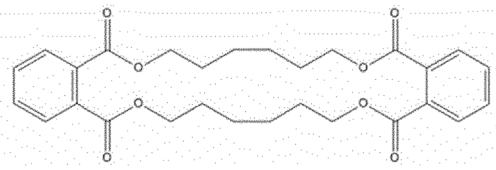

(II)

or a pharmacologically acceptable salt thereof.

The food or beverage for immunostimulation according to the second aspect of the present invention comprises, as an active ingredient, a compound represented by general formula I:

[Formula 3]

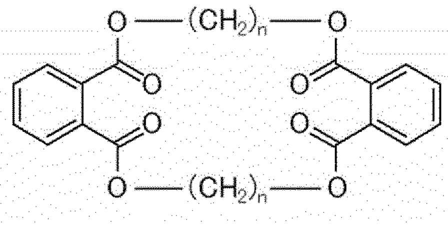

(I)

wherein n represents an integer of 4 to 12,
or a pharmacologically acceptable salt thereof.

For example, the food or beverage for immunostimulation comprises, as an active ingredient, a compound represented by formula II:

[Formula 4]

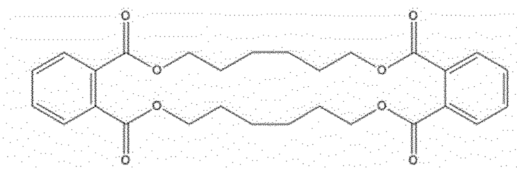

(II)

or a pharmacologically acceptable salt thereof.

Advantageous Effects of Invention

According to the present invention, a novel immunostimulator and a food or beverage for immunostimulation having an excellent immunostimulatory action can be provided.

Brief Description of Drawings

[Figure 1] Figure 1 shows a method for synthesizing AU-1833C.
[Figure 2] Figure 2 shows $^{13}C$ NMR and $^{1}H$ NMR data of the synthesized AU-1833C.
[Figure 3] Figure 3 is a graph showing TNF-α production inducing activities of AU-1833C, Lentinan and Fucoidan.
[Figure 4] Figure 4 shows an immunostimulatory action of AU-1833C, wherein (a) is a graph showing PEC counts, (b) is a graph showing the amount of induced TNF-α production (pg/cell), and (c) is a graph showing the amount of induced TNF-α production (pg/mouse).

Description of Embodiments

First, the immunostimulator of the present embodiment will be described in detail.
The immunostimulator according to the present embodiment comprises, as an active ingredient, a compound represented by general formula I:

[Formula 5]

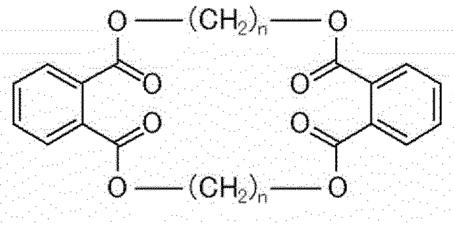

(I)

wherein n represents an integer of 4 to 12,
or a pharmacologically acceptable salt thereof. In the formula, preferably n is 4 to 10, more preferably n is 4 to 8, and further preferably n is 5 to 7.
As used herein, the term "pharmacologically acceptable salt" means a derivative of the disclosed compound, and in that context, the parent compound is modified by an exchange at an acid or base moiety to be a salt. Examples of the pharmacologically acceptable salt include but are not limited to a mineral or organic acid salt of a basic residue such as amine and alkali, and an organic salt of an acid residue such as a carboxylic acid. The pharmacologically acceptable salt herein includes, for example, a conventional non-toxic salt of a parent compound formed from a non-toxic inorganic or organic acid. Herein, the pharmacologically acceptable salt may be synthesized from a parent compound including a base or acid moiety by a conventional chemical method. Typically, such a salt may be prepared by reacting a free acid or free base of the compound with a stoichiometric amount of an appropriate base or acid in water or an organic solvent or in a mixed solution thereof. Generally, nonaqueous media such as ether, ethyl acetate (EtOAc), ethanol, isopropanol, and acetonitrile are preferable. A list of reasonable salts is shown in Remington's Pharmaceutical Sciences, 17th ed., Mack Publishing Company, Easton, Pa., 1985, p.1418 and Journal of Pharmaceutical Science, 66, 2 (1977).
The compound of the general formula I set forth above can be produced by, for example, a known synthesis method such as "Synthesis of New Macrocycles. Part IV. I Two-step Synthesis of Dimeric Phthalic Acid Esters. Journal of the Chemical Society, Perkin Transactions 1, 1974, 2578-2580."
The immunostimulator according to the present embodiment may comprise, as an active ingredient, a compound represented by formula II:

[Formula 6]

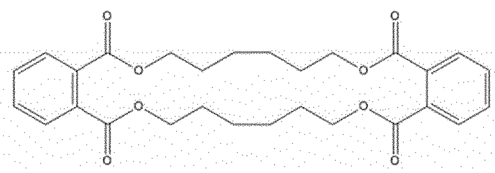

(II)

or a pharmacologically acceptable salt thereof. The compound of formula II is a compound wherein n is 6 in the formula

I described above. The chemical name for the compound of formula II is 7,8,9,10,11,12,19,20,21,22,23,24-dodecahydrodibenzo[1,6,13,18]-tetraoxacyclooctadecine-2,5,14,17-tetrone. Herein, the compound of formula II is sometimes referred to as "AU-1833C."

An example of the method for synthesizing the compound of formula II (AU-1833C) set forth above will be described (Figure 1). Hereinbelow, Compounds 1 to 5 are as shown in Figure 1. Compound 1 is dissolved in pyridine, Compound 2 is added thereto and the resultant is stirred for a specified time at a specified temperature. Compound 1 is further added and the resultant is stirred for a specified time at a specified temperature. Then, after dilution with EtOAc, an HCl solution, a saturated sodium hydrogen carbonate solution, and a saturated sodium chloride solution are added in this order for separation. The EtOAc layer is dried by adding $Na_2SO_4$ and then concentrated to obtain Compound 3. Compound 3, 4-dimethylaminopyridine (DMAP), and N,N'-dicyclohexylcarbodiimide (DCC) are dissolved in anhydrous dichloromethane, and Compound 4 is added thereto and the resultant is stirred for a specified time. Next, after dilution with chloroform, an HCl solution, a saturated sodium hydrogen carbonate solution, and a saturated sodium chloride solution are added in this order for separation. The chloroform layer is dried by adding $Na_2SO_4$, then concentrated, and purified using a silica gel column with EtOAc and hexane thereby to obtain Compound 5. Compound 5 and a specified catalyst are dissolved in anhydrous dichloromethane and stirred for a specified time. Next, purification is carried out using a silica gel column with EtOAc and hexane thereby to obtain Compound 6. Compound 6 is dissolved in methanol, a palladium-activated carbon ethylenediamine complex is added thereto and the resultant is stirred for a specified time in a hydrogen atmosphere. Next, after celite filtration, purification is carried out using a silica gel column with EtOAc and hexane thereby to obtain AU-1833C. Note that the compound of formula II (AU-1833C) set forth above can be synthesized by a method other than this, or can be isolated by processing, extracting and the like of an animal or a plant.

The immunostimulatory action of the immunostimulator according to the present embodiment can be determined to be present, when, for example, the degree of cytokine production enhancement (for example, tumor necrosis factor (TNF-$\alpha$) production enhancement) in mammalian cells to which the immunostimulator is administered is higher than that in non-administered mammalian cells, or the degree of an increase in the peritoneal exudate cell (PEC) count in a mammal to which the immunostimulator is administered is higher than that in a non-administered mammal. Examples of the mammal mentioned above include humans, mice, monkeys, horses, and cows.

The immunostimulator according to the present embodiment can be prepared by a common method into a dosage form such as a tablet, a granule, a powder, a capsule, a syrup, and an injection, and an appropriate drug delivery system (DDS) can also be used. Additionally, an excipient, a binder, a lubricant, a colorant, a disintegrator, a thickener, a preservative, a stabilizer, and a pH adjusting agent typically used in pharmaceutical products can be added. Additionally, the dose of the immunostimulator according to the present embodiment can be suitably determined in accordance with the age, body weight, symptoms to be applied, and the like of a subject. Additionally, the administration method of the immunostimulator according to the present embodiment can be any of administrations with a meal, after a meal, before a meal, between meals, and at bedtime.

Next, the food or beverage for immunostimulation according to the present embodiment will be described.

The food or beverage for immunostimulation according to the present embodiment comprises, as an active ingredient, a compound represented by general formula I:

[Formula 7]

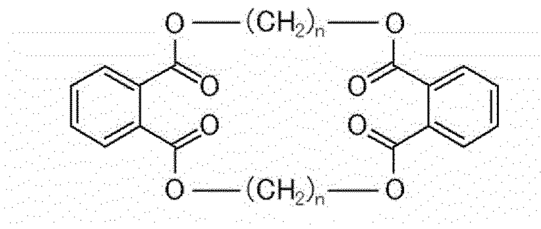

(I)

wherein n represents an integer of 4 to 12,

or a pharmacologically acceptable salt thereof. In the formula, preferably n is 4 to 10, more preferably n is 4 to 8, and further preferably n is 5 to 7.

The food or beverage for immunostimulation according to the present embodiment can comprise, as an active ingredient, for example, a compound represented by formula II:

[Formula 8]

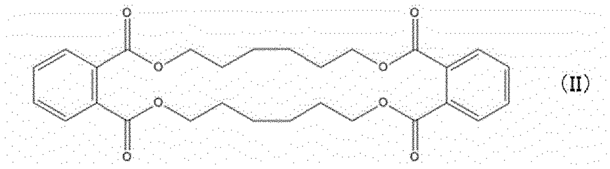

(II)

or a pharmacologically acceptable salt thereof. The compound of formula II is a compound wherein n is 6 in formula I set forth above. The chemical name for the compound of the formula II is 7,8,9,10,11,12,19,20,21,22,23,24-dodecahydro-dibenzo[1,6,13,18]-tetraoxacyclooctadecine-2,5,14,17-tetrone. Herein, the compound of formula II is sometimes referred to as "AU-1833C."

For the food or beverage for immunostimulation according to the present embodiment, details of the "pharmacologically acceptable salt" and "immunostimulatory action" are the same as described above.

The food or beverage for immunostimulation according to the present embodiment can be processed by a common method into a form suitable for eating and drinking such as a granular form, a grain form, a tablet, a capsule, a gel form, a cream form, a paste form, a suspension form, an aqueous solution form, an emulsion form, and a powder form. Additionally, an excipient, a binder, a lubricant, a colorant, a disintegrator, a thickener, a preservative, a stabilizer, and a pH adjusting agent typically used in foods and drinks can be added. Further, for the improvement of taste qualities, saccharides, sugar alcohols, salts, fats and oils, amino acids, organic acids, and glycerin can be added in the range not affecting the effects of the present invention. Note that when the food or beverage for immunostimulation according to the present embodiment contained in an existing food or drink is used, the food or drink to be the base can be suitably selected as long as such a food or drink can provide the effects of the present invention.

The food or beverage for immunostimulation according to the present embodiment can be used as, for example, a food for specified health use, a food with nutrient function claims, a food with functional claims, a supplement, a so-called energy drink, livestock feed and the like.

Examples

Hereinafter, the present invention will specifically be described in reference to Examples. However, the present invention is not limited to these Examples.

(Example 1)

As shown in Figure 1, the compound of formula II (AU-1833C) was synthesized. Compounds 1 to 6 are as shown in Figure 1.

(Synthesis of Compound 5)

3 g (20 mmol) of Compound 1 (manufactured by FUJIFILM Wako Pure Chemical Corporation) was dissolved in 40 mL of pyridine (manufactured by FUJIFILM Wako Pure Chemical Corporation), 1.9 g (16 mmol) of Compound 2 (manufactured by FUJIFILM Wako Pure Chemical Corporation) was added thereto and the resultant was stirred at 80°C for 4 hours. Further, 3 g (20 mmol) of Compound 1 was added and the resultant was stirred at 80°C for 20 hours. Next, after dilution with EtOAc, 1 M of an HCl solution, a saturated sodium hydrogen carbonate solution, and a saturated sodium chloride solution were added in this order for separation. The EtOAc layer was dried by adding $Na_2SO_4$ and then concentrated to obtain Compound 3 (5 g,12 mmol, yield 63%). Compound 3 (5 g, 12 mmol), 1.2 g (1 mmol) of 4-dimethylaminopyridine (DMAP; manufactured by FUJIFILM Wako Pure Chemical Corporation), and 10.3 g (5 mmol) of N,N'-dicyclohexylcarbodiimide (DCC; manufactured by FUJIFILM Wako Pure Chemical Corporation) were dissolved in anhydrous dichloromethane (70 mL), 2.2 g (30 mmol) of Compound 4 (manufactured by FUJIFILM Wako Pure Chemical Corporation) was added thereto and the resultant was stirred overnight. Next, after dilution with chloroform, 1 M of an HCl solution, a saturated sodium hydrogen carbonate solution, and a saturated sodium chloride solution were added in this order for separation. The chloroform layer was dried by adding $Na_2SO_4$, then concentrated and purified using a silica gel column (200 g) with EtOAc:hexane = 40:60 (v/v) thereby to obtain Compound 5 (3.5 g, 6.7 mmol, yield from Compound 3 was 56%). The $^1$H-NMR analysis result for Compound 5 is shown below; $^1$H-NMR (CDCl $_3$, 270 MHz) δ 7.68 (4H, dd, J = 5.7, 3.5 Hz), δ 7.49 (4H, dd, J = 5.4, 3.2 Hz), δ 5.80 (2H, m), δ 5.10 (4H, m), δ 4.30 (8H, m), δ 2.46

(4H, m), δ 1.73 (4H, m), δ 1.49 (4H, m).

(Synthesis of Compound 6)

Compound 5 (3.5 g, 6.7 mmol) and 150 mg (177 mmol) of Grubbs catalyst 2nd generation (manufactured by Sigma-Aldrich Co. LLC) were dissolved in anhydrous dichloromethane (100 mL) and the resultant was stirred overnight. Next, purification was carried out using a silica gel column (200 g) with EtOAc:hexane = 35:65 (v/v) thereby to obtain Compound 6 (1.3 g, 2.6 mmol, yield from Compound 5 was 39%). The $^{1}$H-NMR analysis result for Compound 6 is shown below; $^{1}$H NMR ($CDCl_3$, 270 MHz) δ 7.72 (4H, m), δ 7.53 (4H, m), δ 5.59 (2H, m), δ 4.30 (8H, m), δ2.46 (4H, m), δ 1.75 (4H, m), δ 1.46 (4H, m).

(Synthesis of AU-1833C)

Compound 6 (1.3 g, 2.6 mmol) was dissolved in methanol (60 mL), 60 mg of a palladium-activated carbon ethylenediamine complex (manufactured by FUJIFILM Wako Pure Chemical Corporation) was added thereto and the resultant was stirred for 3 hours in a hydrogen atmosphere. Next, after celite filtration, purification was carried out using a silica gel column (100 g) with EtOAc:hexane = 40:60 (v/v) thereby to obtain AU-1833C (0.9 g, 1.8 mmol, yield from Compound 6 was 68%.)

[Formula 9]

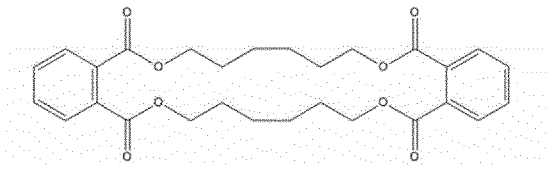

(II)

$^{13}$C NMR (100 MHz) data and $^{1}$H NMR (400 MHz) data of the synthesized compound are shown in Figure 2 (solvent: MeOH-$d_4$). These data revealed that the synthesized compound was definitely the compound of formula (II) (AU-1833C).

(Example 2)

An immunostimulatory action of AU-1833C was investigated.

(TNF-α production-inducing activity in cells)

A TNF-α production-inducing activity in a mouse-derived macrophage-like cell line (RAW264, provided: RIKEN BioResource Research Center) was investigated. Using, as test samples, AU-1833C (synthesized in Example 1), lentinan (product name: Lentinan, manufactured by FUJIFILM Wako Pure Chemical Corporation) and fucoidan (product name: Fucoidan *Fucus vesiculosus*-derived, manufactured by Sigma-Aldrich Co. LLC), test sample solutions were prepared so that the final concentrations of the test samples were 50, 100, and 200 μg/mL. First, all test samples were dissolved in dimethylsulfoxide (DMSO) so as to have a 1,000-fold concentration of the final concentration, and then 100-fold dilution was carried out with deionized water thereby to prepare test sample solutions having a 10-fold concentration (including 1% DMSO) of the final concentrations. RAW264 cells were suspended in DMEM medium (product name: Dulbecco's Modified Eagle Medium "Nissui" 2 powder, manufactured by NISSUI PHARMACEUTICAL CO., LTD.) to which 10% fetal bovine serum (FBS) was added, seeded in a 96-well microplate in a cell density of 10,000 cells/well/100 μL, and cultured at 37°C for 16 hours under 5%$CO_2$. The medium was replaced with fresh DMEM medium (180 μL), and the test sample solution in an amount of one tenth (20 μL) as each test sample group or a 1% DMSO aqueous solution as a non-treated group was added and the cells were cultured at 37°C for 24 hours under 5%$CO_2$. Thereafter, the culture supernatant was collected and subjected to an evaluation test of the TNF-α production-inducing activity. The concentration measurement of the produced TNF-α was carried out using an ELISA kit (product name: Mouse TNF-alpha Quantikine ELISA Kit, manufactured by R&D systems) in accordance with protocol. A ratio of a TNF-α concentration in each test sample group relative to a TNF-α concentration in the non-treated group was calculated from the measured TNF-α concentrations and defined as the TNF-α production-inducing activity.

The results of TNF-α production-inducing activity are shown in Figure 3. At all concentrations of 50, 100, and 200 μg/mL, the TNF-α production-inducing activity of AU-1833C was revealed to be significantly higher than that of the control non-treated group. Additionally, the TNF-α production-inducing activity of AU-1833C at each concentration was revealed to be significantly higher than those of lentinan and fucoidan at the same concentration.
The above revealed that AU-1833C had a significantly higher TNF-α production-inducing activity than lentinan and fucoidan which are the existing immunostimulators, thereby revealing that AU-1833C had an excellent immunostimulatory action.

(PEC count and the amount of induced TNF-α production in mice)

Next, peritoneal exudate cell (PEC) counts and the amount of induced TNF-α production in mice were investigated.
The test sample AU-1833C was dissolved in DMSO so as to be 50 mg/mL and then diluted five-fold with saline (product name: Otsuka normal saline, manufactured by Otsuka Pharmaceutical Co., Ltd.) to prepare a test sample solution of 10 mg/mL (including 20% DMSO).
The animals used were Slc: ddY mice (female, 6-week old) and grouped as follows.

1) Non-treated group: n = 6
2) AU-1833C group: n = 6

The mice purchased at the age of 5 weeks were acclimated for 5 days under a condition of free access to water and feed (regular diet (product name: CE-2, manufactured by CLEA Japan, Inc.)). Body weights of the mice after acclimation at the age of 6 weeks were measured and the mice were grouped based on the measured values so that there was no difference in the body weight among the groups. 200 μL of the test sample solutions or 20% DMSO saline as the non-treated group was respectively administered to the mice by intraperitoneal injection. The mice after a lapse of 22 hours from administration were sacrificed under isoflurane anesthesia, 5 mL of saline was intraperitoneally injected, the abdominal area was rubbed about 10 times, and then intraperitoneal cells were collected by collecting the saline from the abdominal cavity. Using a portion of the collected saline and a Turk's solution (product name: Turk's solution, manufactured by Wako Pure Chemical Industries, Ltd.), a concentration of PEC (cells/mL), intraperitonially exudated nucleated cells such as white blood cells, was measured. The measured PEC concentration was multiplied by the amount of saline intraperitoneally injected (5 mL) to calculate a PEC count per mouse (cells/mouse).
The remaining of the collected saline was centrifuged to collect PECs for measuring the TNF-α production-inducing ability of PEC when treated with lipopolysaccharide (LPS) (product name:
lipopolysaccharide from *Escherichia coli* O111: B4, manufactured by Sigma-Aldrich Co. LLC). PECs were suspended in serum free RPMI 1640 medium (product name: RPMI 1640 medium "Nissui" 2 powder, manufactured by NISSUI PHARMACEUTICAL CO., LTD.), and then washed by centrifugation again and suspended in RPMI 1640 medium to which 10% fetal bovine serum (FBS) was added. Using a Turk's solution, the cell count after wash was calculated again, then the concentration was adjusted with serum-containing RPMI 1640, and the cells were seeded in a 96-well microplate in a cell density of 800,000 cells/well/180 μL. LPS was added to make a total medium volume of 200 μL in such a way that the final concentration was 100 ng/mL, and the cells were cultured for 3 hours at 37°C under 5%$CO_2$. Thereafter, the culture supernatant was collected and subjected to an evaluation test of the amount of induced TNF-α production. The measurement of a TNF-α concentration was carried out using an ELISA kit in accordance with the protocol. From the measured TNF-α concentration (pg/1,000 μL), a TNF-α amount per cell was calculated using the following equation 1, and a TNF-α amount produced by PEC per mouse was calculated using the following equation 2.

Equation 1

TNF-α concentration (pg/1,000 μL) × (200 μL/1,000)/800,000 cells = TNF-α production amount (pg/cell)

Equation 2

$$\text{TNF-}\alpha\text{ concentration (pg/1,000 }\mu\text{L)} \times \text{(200 }\mu\text{L/1,000)/800,000 cells} \times \text{PEC count per mouse (cells/mouse)} = \text{TNF-}\alpha\text{ production amount (pg/mouse)}$$

The results are shown in Figure 4. It was revealed that the mice to which AU-1833C was intraperitoneally administered had significantly higher PEC counts (Figure 4(a)) as compared with the control non-treated mice. Additionally, it was revealed that the mice to which AU-1833C was administered had a significantly higher TNF-α production amount per cell in PEC (Figure 4(b)) and also had a significantly higher PEC-derived TNF-α production amount per mouse (Figure 4(c)) than the control non-treated mice.

The above revealed that the AU-1833C administration increased the PEC count, increased the amount of induced TNF-α production in PECs, and increased the amount of PEC-derived induced TNF-α production per mouse, thereby revealing that AU-1833C had an excellent immunostimulatory action.

Note that the present invention can be carried out in various embodiments and modifications without departing from the broad spirit and scope of the present invention. Additionally, the embodiments described above are for the purpose of describing the present invention and do not intend to limit the scope of the present invention. That is, the scope of the present invention is defined by the claims, but not by the embodiments. Thus, various modifications carried out within the claims and within the scope of meaning of the invention equivalent thereto are considered within the scope of the present invention.

The present invention is based on Japanese Patent Application No. 2019-67663 filed on March 29, 2019. The description, the claims, and all the drawings disclosed in Japanese Patent Application No. 2019-67663 shall be incorporated herein by reference in its entirety.

## Claims

1. An immunostimulator comprising, as an active ingredient, a compound represented by general formula I:

[Formula 1]

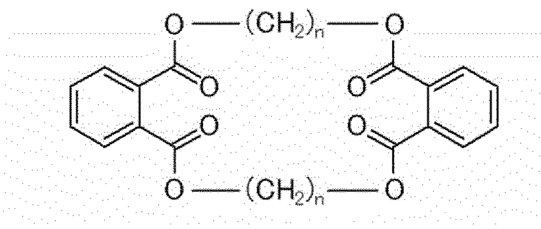

(I)

wherein n represents an integer of 4 to 12,
or a pharmacologically acceptable salt thereof.

2. The immunostimulator according to claim 1, comprising, as an active ingredient, a compound represented by formula II:

[Formula 2]

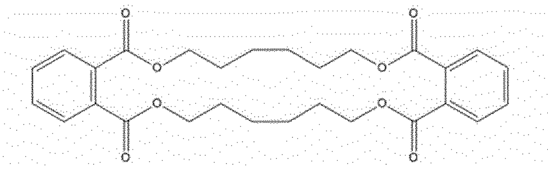

(II)

or a pharmacologically acceptable salt thereof.

**3.** A food or beverage for immunostimulation comprising, as an active ingredient, a compound represented by general formula I:

[Formula 3]

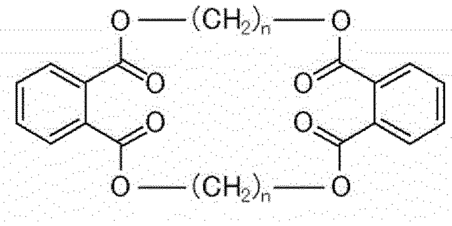

(I)

wherein n represents an integer of 4 to 12,
or a pharmacologically acceptable salt thereof.

**4.** The food or beverage for immunostimulation according to claim 3 comprising, as an active ingredient, a compound represented by formula II:

[Formula 4]

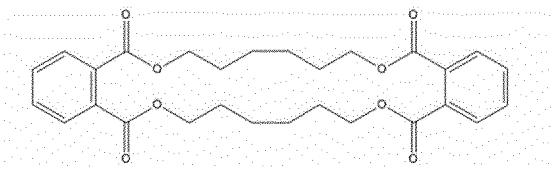

(II)

or a pharmacologically acceptable salt thereof.

Fig. 1

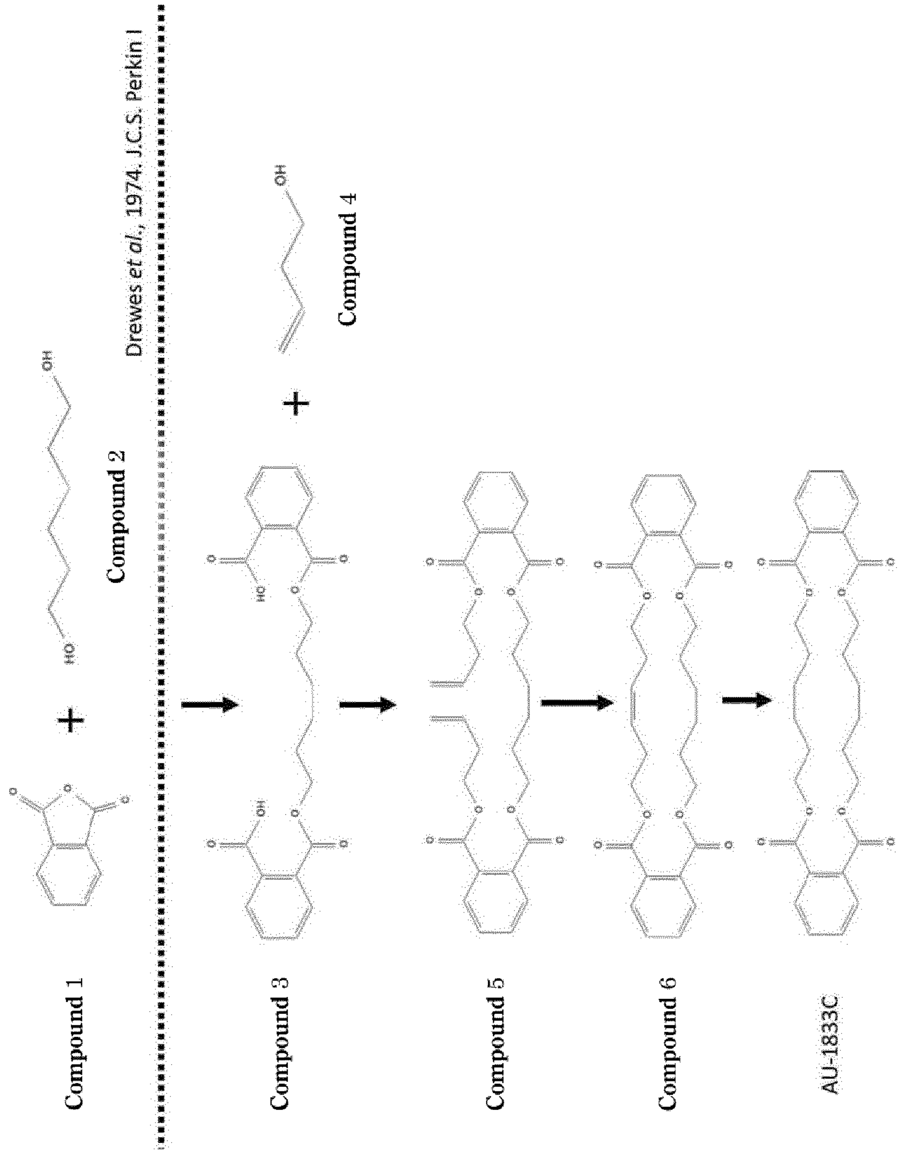

Compound 1

Compound 2

Drewes *et al.*, 1974. J.C.S. Perkin I

Compound 3

Compound 4

Compound 5

Compound 6

AU-1833C

Fig. 2

$^{13}C$ NMR (100 MHz) and $^{1}H$ NMR (400 MHz) data (solvent: MeOH-$d_4$)

| Position | $\delta_C$ | $\delta_H$ |
|---|---|---|
| 1 | 132.4 | 7.60 (1H, dd, *J* = 5.9 Hz, 3.4 Hz) |
| 2 | 129.9 | 7.71 (1H, dd, *J* = 5.9 Hz, 3.4 Hz) |
| 3 | 133.6 | - |
| 4 | 169.4 | - |
| 5 | 66.9 | 4.29 (2H, t, *J* = 6.6 Hz) |
| 6 | 29.7 | 1.76 (2H, m) |
| 7 | 26.9 | 1.47 (2H, m) |

Fig. 3

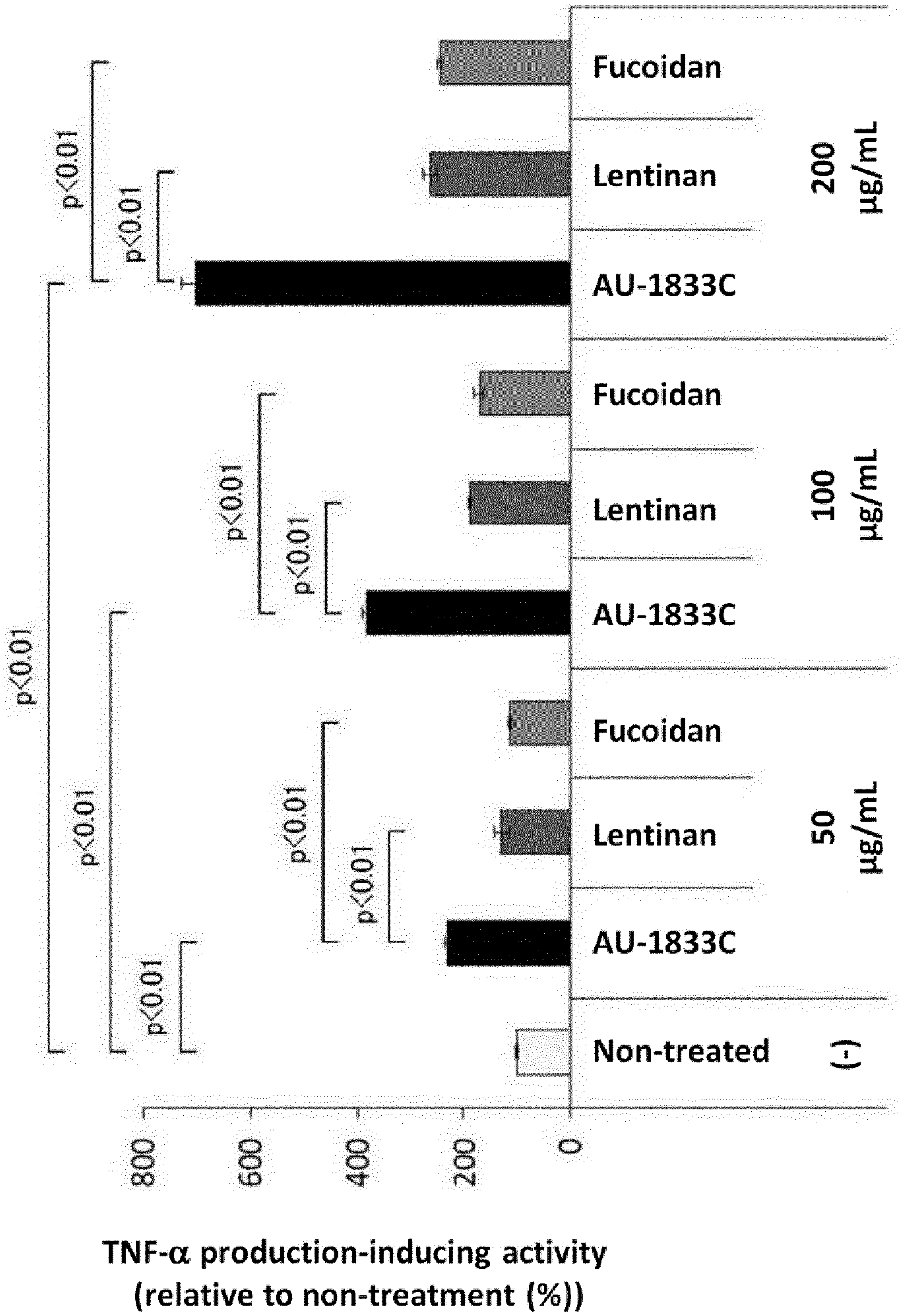
Fucoidan
Lentinan
AU-1833C
200 µg/mL
Fucoidan
Lentinan
AU-1833C
100 µg/mL
Fucoidan
Lentinan
AU-1833C
50 µg/mL
Non-treated
(-)
p<0.01
p<0.01
p<0.01
p<0.01
p<0.01
p<0.01
p<0.01
p<0.01
p<0.01
800
600
400
200
0
TNF-α production-inducing activity
(relative to non-treatment (%))

Fig. 4

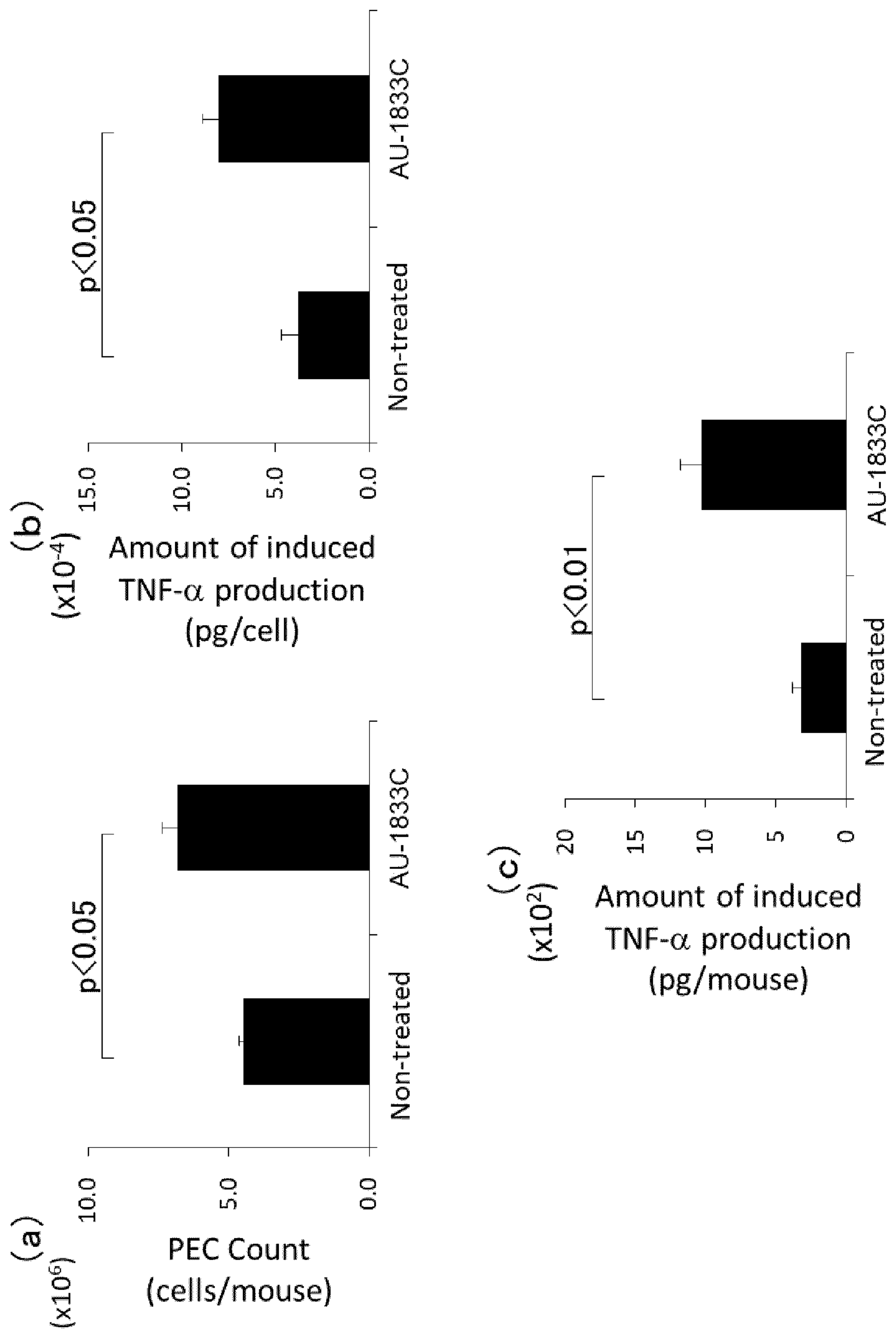
(a)
(x10^6)
PEC Count
(cells/mouse)
10.0
5.0
0.0
Non-treated
AU-1833C
p<0.05
(b)
(x10^-4)
Amount of induced
TNF-α production
(pg/cell)
15.0
10.0
5.0
0.0
Non-treated
AU-1833C
p<0.05
(c)
(x10^2)
Amount of induced
TNF-α production
(pg/mouse)
20
15
10
5
0
Non-treated
AU-1833C
p<0.01

## INTERNATIONAL SEARCH REPORT

International application No. PCT/JP2020/013504

**A. CLASSIFICATION OF SUBJECT MATTER**

Int. Cl. A61P37/04(2006.01)i, A23L33/10(2016.01)i, A61K31/357(2006.01)i
FI: A23L33/10, A61K31/357, A61P37/04

According to International Patent Classification (IPC) or to both national classification and IPC

**B. FIELDS SEARCHED**

Minimum documentation searched (classification system followed by classification symbols)
Int. Cl. A23L31/00-33/29, A61K31/00-36/9068, A61P1/00-43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2016-160237 A (HIROSHIMA UNIVERSITY) 05 September 2016, claims 1-6 | 1-4 |
| A | JP 2013-180995 A (NIPPON PAPER GROUP INC.) 12 September 2013, claim 7 | 1-4 |

☐ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 08.05.2020 | 26.05.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/013504

| Patent Documents referred to in the Report | Publication Date | Patent Family | Publication Date |
|---|---|---|---|
| JP 2016-160237 A | 05.09.2016 | (Family: none) | |
| JP 2013-180995 A | 12.09.2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016160237 A
- JP 2013180995 A
- JP 2019067663 A


### Non-patent literature cited in the description


- **GAGOME KOMBU.** *Immune Efficacy and Safety of Fucoidan Extracted*
- **HIROMU ONOGI et al.** *Japanese Journal of Complementary and Alternative Medicine,* 2015, vol. 12 (2), 87-93
- Remington's Pharmaceutical Sciences. Mack Publishing Company, 1985, 1418
- *Journal of Pharmaceutical Science,* 1977, vol. 66, 2
- Synthesis of New Macrocycles. Part IV. I Two-step Synthesis of Dimeric Phthalic Acid Esters. Journal of the Chemical Society. Perkin Transactions, 1974, vol. 1, 2578-2580